# EUROPÄISCHE PATENTANMELDUNG

(11) **EP 2 229 965 A1**
(43) Veröffentlichungstag der Anmeldung: **22.09.2010**
(21) Anmeldenummer: 09075127.2
(22) Anmeldetag: 18.03.2009
(51) Int. Cl.: A61M 1/10, F04D 29/24

(54) **Fluidpumpe mit besonderer Gestaltung eines Rotorblattes**

(71) Anmelder: ECP Entwicklungsgesellschaft mbH, 12247 Berlin (DE)
(72) Erfinder: Röhn, Daniel, 12557 Berlin (DE)
(74) Vertreter: Pfenning, Meinig & Partner GbR

(57) **Zusammenfassung**

Die Erfindung bezieht sich auf eine Fluidpumpe, insbesondere eine Flüssigkeitspumpe, mit einem Rotor, mit einer Rotorwelle (11) und wenigstens einem Rotorblatt zur Förderung des Fluids, wobei der Rotor bezüglich seines Durchmessers zwischen einem ersten, komprimierten Zustand und einem zweiten, expandierten Zustand veränderbar ist, wobei das wenigstens eine Rotorblatt wenigstens zwei entlang der Längsachse der Rotorwelle voneinander beabstandete Aufstellelemente (12,13,14) aufweist, die in expandiertem Zustand des Rotors von der Welle abstehen, sowie wenigstens zwei biegeschlaffe Spannelemente (25,26,27,28,29,30), die mit Abstand voneinander von einem Aufstellelement wenigstens bis zu einem weiteren Aufstellelement verlaufen, wobei zwischen den Spannelementen eine biegeschlaffe Membran (10) gehalten ist, welche in expandiertem Zustand des Rotors gespannt ist. Durch die membranartige Gestaltung der Rotorblätter ist eine gute Komprimierbarkeit des Rotors mit geringen Gegenkräften sowie ein leichtes Aufstellen des Rotors erreichbar. Die Membran kann beispielsweise im Tauchverfahren hergestellt sein.

## Beschreibung

Die Erfindung liegt auf dem Gebiet der Medizintechnik, insbesondere der Feinwerktechnik, wo die präzise Gestaltung und Fertigung von technischen Bauteilen geringer Baugröße im Vordergrund steht.

Dieses Problem spielt insbesondere beim Bau von Mikropumpen eine Rolle, die einerseits hoch leistungsfähig und zuverlässig sein und andererseits bei möglichst geringen Kosten eine minimale Baugröße mit einer akzeptablen Förderleistung verbinden sollen.

Zudem ergibt sich bei vielen medizinischen Anwendungen wie auch bei speziellen anderen Anwendungen das Erfordernis, dass eine derartige Pumpe zur Einführung in einen schwer zugänglichen Raum vorübergehend verkleinert werden kann, um sie nach dem Einbringen in den Einsatzraum wieder entsprechend vergrößern zu können. Im medizinischen Bereich kommt speziell die Forderung hinzu, dass die Pumpe nach dem Einsatz beispielsweise in einem Raum innerhalb eines menschlichen Körpers auch wieder verkleinert werden kann, um sie ohne größere Eingriffe aus dem Körper wieder zu entfernen.

In der Medizintechnik tritt diese Aufgabe beispielsweise bei der Schaffung von Katheterpumpen auf, die in die Blutbahn eingeführt werden können und z. B. in einer Herzkammer Blut fördern.

Derartige, insbesondere in ihrem Durchmesser veränderbare Fluidpumpen sind beispielsweise in Konstruktionen bekannt, die sogenannte Formgedächtniswerkstoffe verwenden, wobei die Konstruktion bei einer ersten Temperatur eine bestimmte Form erhält und diese beim Übergang zu einer anderen Temperatur selbsttätig verändert.

Bei der Verwendung derartiger Materialien ist außer dem hohen Preis der Ausgangsmaterialien jedoch auch eine Reihe von zusätzlichen mechanischen Problemen zu bedenken, die speziell mit den Gedächtniseigenschaften zusammenhängen und funktional nachteilig sein können.

Expandierbare und komprimierbare Fluidpumpen sind aus dem Stand der Technik bereits bekannt. Das Patentdokument DE 100 59 714 C1 zeigt beispielsweise eine Pumpe, die mitsamt dem Pumpenantrieb durch ein Blutgefäß geschoben werden kann. Das Blut fließt dort durch eine Kanüle, deren Durchmesser zur Veränderung der Strömungsverhältnisse expandierbar oder komprimierbar ist.

Eine weitere Konstruktion einer Blutpumpe geht aus der WO 03/103745 A2 hervor, deren Rotor radial komprimierbar und expandierbar ist. Gemäß der Offenlegungsschrift werden verschiedene Konstruktionen zur Erreichung der Expandierbarkeit vorgeschlagen. Beispielsweise kann durch verschiedene, gegeneinander verschiebbare Teile des Katheters nach der Einführung in einen Patientenkörper eine Stauchung des Pumpengehäuses und damit verbunden eine radiale Aufweitung erreicht werden. Zudem ist die Möglichkeit offenbart, durch Drehung einer Antriebswelle gegenüber einem in dem Katheter befindlichen Draht eine Helixstruktur eines Drahtes zu erzeugen, der die radial äußere Begrenzung einer Rotorschaufel bildet.

Die WO 03/103745 A2 zeigt außerdem eine Rotorstruktur mit einer Mehrzahl von in sich steifen, schwenkbar an einem zentralen Teil angelenkten Schaufeln, die sich im Betrieb der Pumpe aufstellen und damit einen Fluiddruck erzeugen.

Aus der EP 0 768 900 B1 ist eine Pumpe bekannt, bei der innerhalb eines Pumpengehäuses an eine Welle Rotorblätter derart angelenkt sind, dass sie im Ruhezustand an die Welle angeklappt und im Betrieb senkrecht zur Welle aufgestellt werden können, um das Fluid zu fördern.

Gemäß dem bekannten Stand der Technik sind somit eine Reihe von Mechanismen bekannt, die ein aktives Aufstellen einer Förderschaufel / eines Rotorblattes mittels Betätigungselementen erlauben, wobei die Fläche des Rotorblattes jeweils verformt und/oder entfaltet wird.

Der vorliegenden Erfindung liegt die Aufgabe zugrunde, eine Fluidpumpe zu schaffen, deren Rotor möglichst einfach aufgebaut ist, um sowohl ein sicheres Funktionieren im Betrieb als auch ein einfaches und zuverlässiges Expandieren sowie Komprimieren mit möglichst geringer Gegenkraft zu erlauben.

Die Aufgabe wird gemäß der Erfindung mit den Merkmalen des Patentanspruchs 1 gelöst.

Dabei liegt der Erfindung der Gedanke zugrunde, dass ein Rotorblatt der Pumpe wenigstens zwei Aufstellelemente aufweist, die in Längsrichtung der Rotorwelle voneinander beabstandet an dieser beweglich befestigt sind und die biegeschlaffe Spannelemente, beispielsweise Spanndrähte oder -bänder tragen, die mit Abstand voneinander zwischen wenigstens zwei Aufstellelementen verlaufen, wobei zwischen den Spannelementen eine biegeschlaffe Membran gehalten ist.

Im komprimierten Zustand des Rotors sind die Aufstellelemente möglichst weit an die Rotorwelle angelegt, so dass die Pumpe auch bei Rotation des Rotors nahezu keine Flüssigkeit fördert. Werden die Aufstellelemente aufgestellt, d. h. wenigstens teilweise von der Rotorwelle wegbewegt, so spannen die zwischen diesen verlaufenden Spannelemente eine Membran auf, die die wesentliche Fläche des Rotorblattes / der Förderschaufel der Pumpe bildet.

Durch die erfindungsgemäße Konstruktion wird das Rotorblatt einerseits im komprimierten Zustand besonders klein, so dass es platzsparend unterbringbar ist, andererseits stellt es im aufgestellten Zustand der Aufstellelemente eine ausreichend große und hinreichend stabilisierte Fläche zur Verfügung, mittels deren beim Betrieb des Rotors das Fluid gefördert werden kann. Die Spannelemente stabilisieren die Membran derart, dass ein Reißen trotz dünnster Ausführung der Membran verhindert ist. Die Spannelemente können die äußere Begrenzung der Membran bilden und damit auch ein Einreißen der Membran von der Außenseite her, beispielsweise aufgrund von Überbelastungen oder mechanischen Verletzungen, verhindern.

Die Spannelemente ihrerseits sind zwischen den Aufstellelementen gehalten, die durch ihre Beweglichkeit das Aufklappen des Rotorblattes erlauben.

Die Aufstellelemente sind beispielsweise jeweils mit einem ersten ihrer Enden mit der Rotorwelle schwenkbar verbunden. Dabei kann jedes Aufstellelement in sich steif und entweder mittels eines Lagers oder eines Filmgelenks mit der Rotorwelle oder einer Nabe verbunden sein.

Durch entsprechende Gestaltung des Gelenks bzw. Filmgelenks oder des Lagers kann gleichzeitig die Schwenkbewegung der Aufstellelemente derart begrenzt werden, dass diese einerseits durch den Fluidgegendruck des zu fördernden Fluids im Betrieb aufgestellt werden, andererseits jedoch ihre Aufstellbewegung im Zustand der größten Expansion des Rotorblattes begrenzt wird, so dass das Rotorblatt im Betrieb stabilisiert ist.

Die Aufstellelemente können in sich steif oder bis zur einer bestimmten Formgrenze auch biegbar sein.

Die Aufstellelemente können gegenüber der Rotorwelle in Umfangsrichtung schwenkbar sein oder auch in einer Ebene, die die Längsachse der Rotorwelle enthält.

In jedem Fall kann die Anlenkung der Aufstellelemente derart gestaltet sein, dass diese im Ruhezustand annähernd parallel zur Rotorwelle an dieser anliegen und im Betriebszustand wenigstens teilweise radial von dieser abgespreizt sind.

Vorteilhaft können die Aufstellelemente eine Form aufweisen, die im komprimierten Zustand bei einer Rotation des Rotors in Betriebsrichtung durch den Fluidgegendruck eine auf die Aufstellelemente wirkende, das Aufstellen der Aufstellelemente bewirkende Kraft erzeugt.

Diese Form kann beispielsweise durch Abschrägungen bzw. eine Schaufelform oder Propellerform der Aufstellelemente erreicht werden.

Die Aufstellelemente können vorteilhaft entlang der Längsachse der Rotorwelle gegeneinander in Umfangsrichtung versetzt sein. Dadurch wird bei zwei oder mehreren zu einer einzigen Rotorschaufel oder einem einzigen Rotorblatt gehörigen Aufstellelementen bezüglich der zwischen ihnen gehaltenen Membran eine helixartige Form ermöglicht.

Die Membran kann vorteilhaft fest mit wenigstens zwei Spannelementen verbunden sein, die beispielsweise als Drähte ausgebildet sein können.

Dabei können die Spannelemente vorteilhaft mit wenigstens zwei Aufstellelementen zugfest verbunden sein, beispielsweise mit den in Längsrichtung der Rotorwelle gesehen äußersten Aufstellelementen.

Die Spannelemente können auch mit den dazwischen liegenden Aufstellelementen, soweit solche existieren, zugfest verbunden sein, jedoch ist auch denkbar, dass die Spannelemente in diesen nur geführt werden und sich zumindest ein Stück weit diesen mittleren Aufstellelementen gegenüber bewegen können.

Durch die Länge der Spannelemente kann damit auch vorbestimmt werden, wie weit die einzelnen Aufstellelemente im Betrieb des Rotors zur Formung einer aufgestellten Membran auslenkbar sind. Die Begrenzung der Bewegung der Aufstellelemente kann somit durch geeignete Wahl der Spannelemente, insbesondere ihrer Länge, gewährleistet werden.

Die Spannelemente können dabei vorteilhaft parallel zueinander, insbesondere wendelförmig um die Rotorwelle herum, verlaufen. Es kann auch vorgesehen sein, dass im Bereich besonders hoher Beanspruchung der Membran im Betrieb die Spannelemente konzentriert sind.

Die Spannelemente können aus demselben Material wie die Membran, beispielsweise einem Kunststoff, bestehen und strangartig ausgebildet sein. Sie können jedoch auch aus einem festeren, weniger dehnbaren Material als die Membran bestehen, beispielsweise einem anderen Kunststoff oder einem Metall. Die Membran ist vorteilhaft elastischer und leichter verformbar gestaltet als die Spannelemente.

Zur Erzeugung eines möglichst großen Pumpendrucks ist vorteilhaft vorgesehen, dass die Membran an der Rotorwelle dicht abschließt. Dadurch wird das Überströmen von Fluid an der Membran vorbei im Bereich der Rotorwelle verhindert, das zu Druckverlust und Leistungsverlust der Pumpe führt.

Die Erfindung bezieht sich außer auf eine Fluidpumpe der oben erläuterten Art mit einer entsprechenden Gestaltung des Rotors auch auf ein Verfahren zur Herstellung eines Rotors für eine derartige Pumpe, bei dem durch Eintauchen der Spannelemente in eine Flüssigkeit zwischen diesen eine flüssige Membran gebildet wird, die sich nach dem Entfernen aus der Flüssigkeit verfestigt.

Durch dieses Fertigungsverfahren lässt sich die Membran einfach herstellen und im selben Arbeitsgang auch mit den Spannelementen fest verbinden. Im gleichen Zuge kann zumindest in zwei Dimensionen die gewünschte Verteilung der Spannelemente auf die Membranfläche festgelegt werden.

Selbstverständlich kann die Membran auch durch Aufkleben einer vorgefertigten Folie auf die Spannelemente oder durch eine ähnliche Fügetechnik hergestellt werden.

Im Folgenden wird die Erfindung anhand eines Ausführungsbeispiels anhand mehrerer Zeichnungen gezeigt und anschließend beschrieben. Dabei zeigt
- Fig. 1: in einem Schnitt eine Anwendung eines mit einer Pumpe versehenen Katheters,
- Fig. 2: beispielhaft einen Pumpenrotor mit einem Rotorblatt,
- Fig. 3: eine Rotorwelle sowie ein Aufstellelement,
- Fig 4: in einer Draufsicht eine Rotorwelle mit ei- ner Nabe und zwei Rotorblättern,
- Fig. 5: eine Queransicht des Rotors aus Fig. 4,
- Fig. 6: schematisch den Herstellvorgang für ein Ro- torblatt mit einem Tauchverfahren zur Her- stellung einer Membran,
- Fig. 7: eine dreidimensionale Ansicht eines Pumpen- rotors in expandierter Form,
- Fig. 8: eine dreidimensionale Ansicht eines Pumpen- rotors in komprimierter Form mit angeklapp- ten Aufstellelementen,
- Fig. 9: ein einzelnes Aufstellelement,
- Fig. 10: ein weiteres Beispiel eines Pumpenrotors in expandierter Form,
- Fig. 11: einen Pumpenrotor in expandierter Form, wo- bei nur die Hälfte der Aufstellelemente aufgestellt ist,
- Fig. 12: in einer Draufsicht einen Rotor mit zwei profilierten Aufstellelementen,sowie
- Figur 13: einen Rotor mit aufgestellten, propellerar- tigen Aufstellelementen, jedoch ohne Memb- ran, in perspektivischer Ansicht.

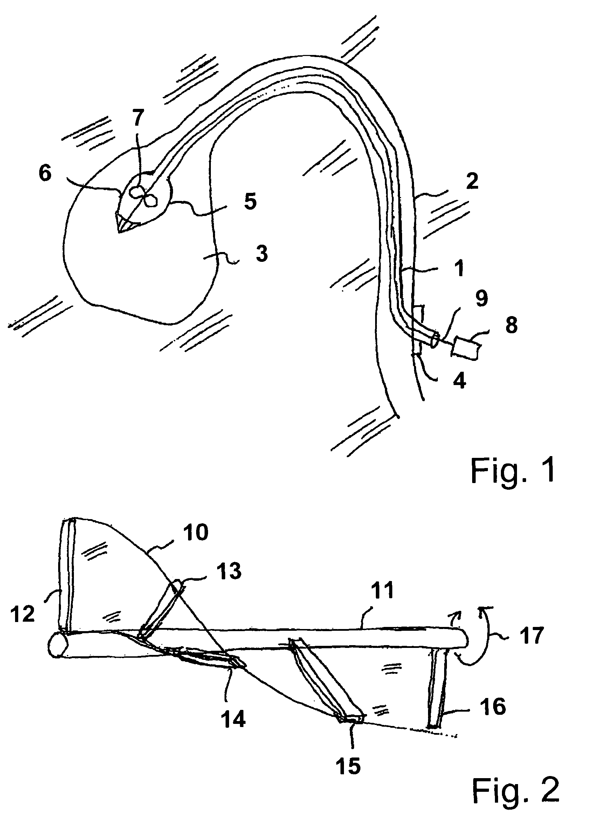

Fig. 1 zeigt einen Katheter 1, der in ein menschliches Blutgefäß 2 bis in eine Herzkammer 3 eingeführt ist und durch eine Schleuse 4 aus dem Gefäß 2 herausragt. Durch diese Schleuse ist der Katheter zusammen mit der Mikropumpe 5 in die Herzkammer 3 eingeführt worden. Zu diesem Zweck ist beim Einführen die Pumpe in einen komprimierten Zustand versetzt, in dem sowohl das Pumpengehäuse 6 als auch der Rotor 7 bezüglich ihres Durchmessers verkleinert sind. Die Konstruktion des Rotors und des Pumpengehäuses sieht vor, dass keine großen Rückstellkräfte entstehen, die das Bestreben haben, die Pumpe gegen die Kraft der Gefäßwände zu expandieren.

Sobald die Pumpe 5 in die Herzkammer 3 eingeführt ist, kann durch den Motor 8 mittels der durch den Katheter 1 geführten Welle 9 der Rotor 7 in Drehung versetzt werden, um Flüssigkeit zu fördern. Gleichzeitig stellt sich wenigstens ein Rotorblatt 10, wie in Fig. 2 dargestellt, auf, entweder durch eine mechanische Betätigung oder durch die eigene Rotation und die Wirkung des Fluidgegendrucks bei der Inbetriebnahme der Pumpe. Auf die entsprechende Formgebung und die Mechanismen wird weiter unten im Einzelnen noch näher eingegangen.

Die Fig. 2 zeigt in einer dreidimensionalen Darstellung eine Rotorwelle 11 mit Aufstellelementen 12, 13, 14, 15, 16 sowie eine zwischen den Aufstellelementen 12 bis 16 gespannte Membran 10, die die Rotorschaufel bzw. das Rotorblatt bildet. Die Membran 10 bildet in voll aufgestelltem Zustand eine helixartige Struktur, so dass der Pumpenrotor bei Rotation in Axialrichtung Flüssigkeit fördert. Die Drehrichtung der Welle ist durch den Pfeil 17 angedeutet.

Die Aufstellelemente 12 bis 16 sind gemäß der Erfindung mit der Rotorwelle 11 beweglich verbunden und können im Ruhezustand, d. h. im komprimierten Zustand, an diese angeklappt werden.

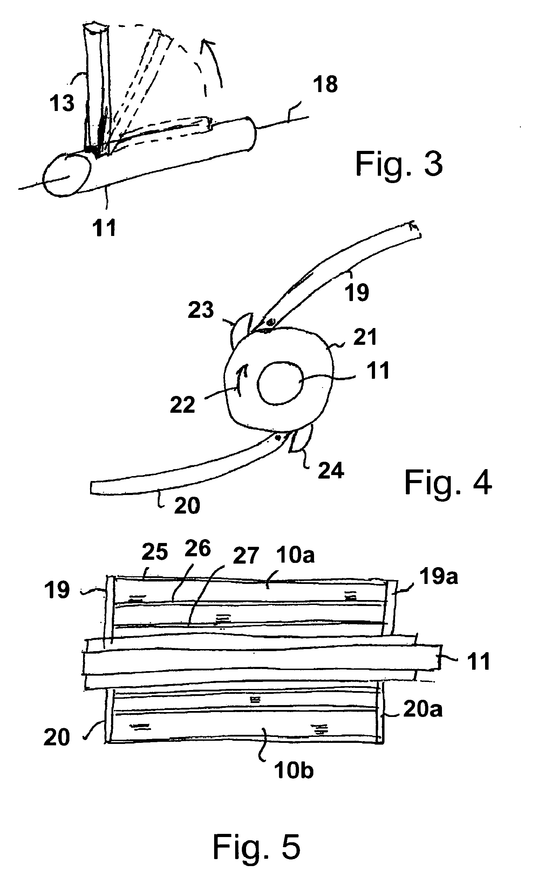

Fig. 3 zeigt hierzu beispielsweise den Fall, dass das Aufstellelement 13 innerhalb einer Ebene klappbar ist, die die Längsachse 18 der Rotorwelle 11 enthält. Verschiedene mögliche Positionen des Aufstellelements 13 sind gestrichelt dargestellt. Das Aufstellelement 13 kann an der Welle 11 mittels eines Filmgelenkes oder mittels eines Lagers schwenkbar befestigt sein.

Die Fig. 4 zeigt eine andere Art der Gestaltung der Aufstellelemente 19, 20 in einer Draufsicht auf die Welle 11, die eine Nabe 21 trägt.

Wird der Rotor in Richtung des Pfeils 22 gedreht, so werden die Aufstellelemente 19, 20 durch den Fluidgegendruck weiter aufgestellt, bis sie an die Anschläge 23, 24 stoßen. In dieser Position stellt der Rotor dem Fluid den größten Widerstand entgegen.

Zu den Aufstellelementen 19, 20 ist jeweils wenigstens ein weiteres Aufstellelement 19a, 20a vorgesehen, zwischen denen Spannelemente 25, 26, 27 befestigt sind. Zwischen den Spannelementen 25, 26, 27 ist eine Membran 10a, 10b befestigt (Fig 5).

Sind die Aufstellelemente 19, 19a und 20, 20a in Umfangsrichtung der Welle 11 nicht gegeneinander versetzt, so kann der dargestellte Rotor für eine Radialpumpe verwendet werden.

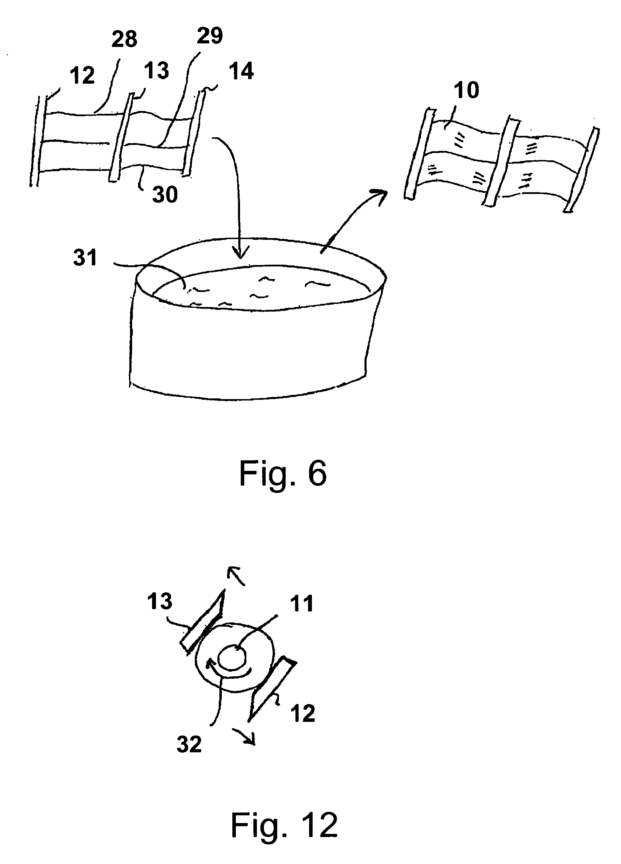

Sind die Aufstellelemente 19, 19a gegeneinander in Umfangsrichtung der Welle 11 versetzt, so nimmt die jeweilige Membran 10a, 10b eine helixartige Form an, und der Pumpenrotor kann auch zur wenigstens teilweise axialen Förderung eines Fluids genutzt werden. Anhand der Fig. 6 ist die mögliche Herstellung eines Rotorblattes dargestellt, wobei von drei Aufstellelementen 12, 13, 14 ausgegangen wird, zwischen denen Spannelemente 28, 29, 30 in Form von Drähten oder Kunststoffsträngen befestigt sind. Dieses vorgefertigte Teil wird in eine Flüssigkeit 31 eingetaucht, die beispielsweise aus einem Harz bestehen oder ein Harz enthalten kann.

Die Viskosität der Flüssigkeit 31 und die Abstände zwischen den Spannelementen 28, 29, 30 sind derart eingestellt, dass sich durch Oberflächenspannung ein Flüssigkeitsfilm zwischen den Spannelementen bildet, der eine Zeitlang stabil ist, bis die Flüssigkeit durch Aushärten oder Abkühlen erhärtet und verfestigt ist.

Die Zahl der Spannelemente kann je nach Bedarf statt drei auch zwei oder mehr als drei betragen.

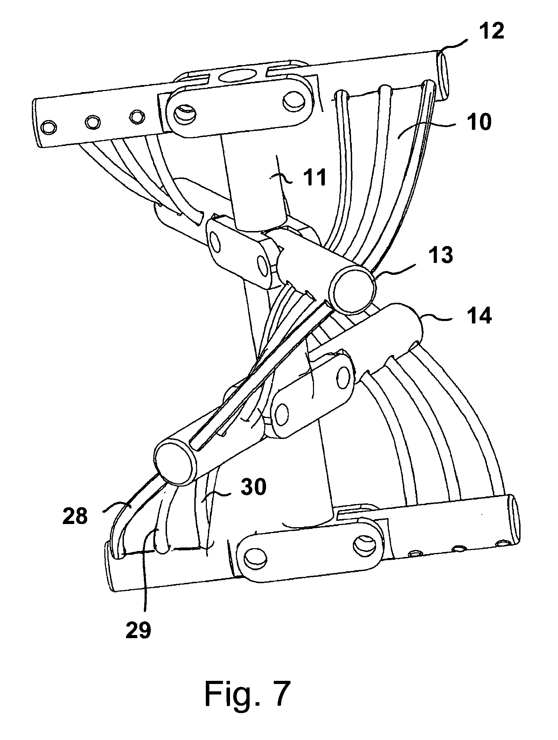

Die Fig. 7 zeigt eine dreidimensionale Ansicht eines Pumpenrotors mit einer Welle 11, von der drei Aufstellelemene 12, 13, 14 maximal im rechten Winkel abstehen.

Zwischen den Aufstellelemente 12, 13, 14 sind Spannelemente 28, 29, 30 befestigt, zwischen denen eine Membran 10 gebildet ist. Diese nimmt, ähnlich wie die Spannelemente, durch die Verdrehung der Aufstellelemente 12, 13, 14 eine helixartige Struktur an, die bei Rotation des Pumpenrotors zu einer axialen Förderung eines Fluids führt.

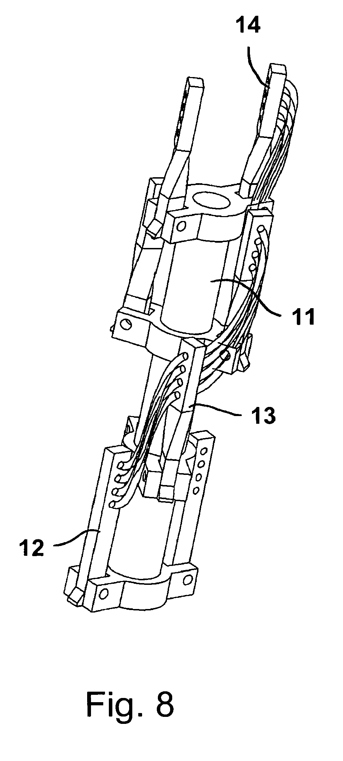

In der Fig. 8 ist der Rotor aus der Fig. 7 in komprimierter Form dargestellt, wobei die Aufstellelemente 12, 13, 14 sowie die jeweils diesen gegenüberliegenden, in der Fig. 7 nicht näher bezeichneten Aufstellelemente jeweils parallel zu der Welle 11 angeklappt sind. Die Spannelemente verlaufen in diesem Zustand ebenfalls parallel zu der Rotorwelle 11, und die Membran setzt einer Rotation in diesem Zustand kaum Widerstand entgegen.

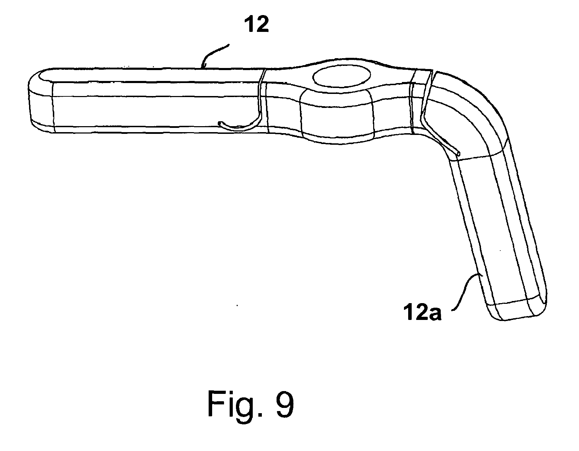

Die Fig. 9 zeigt in einem Detail zwei Aufstellelemente 12, 12a, die einander im eingebauten Zustand an einer Welle 11 diametral gegenüberliegen und die um 90° abklappbar sind.

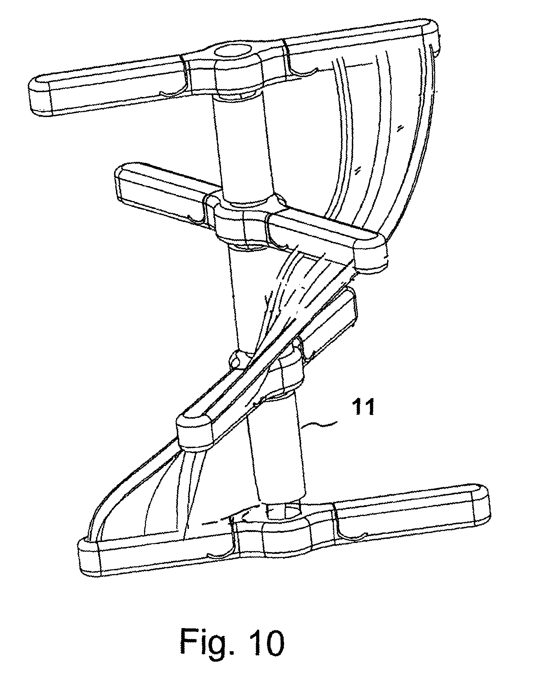

Fig. 10 zeigt acht derartige Aufstellelemente, von denen jeweils zwei einander an der Welle 11 diametral gegenüberliegen und wobei nur zwischen vieren der Aufstellelemente entsprechende Spannelemente mit einer Membran befestigt sind. Die Membran verläuft helixartig und liegt im Bereich der Welle an dieser an. Um den Wirkungsgrad der Pumpe zu erhöhen, werden auch die übrigen Aufstellelemente vorteilhaft noch mit einer Membran versehen, die ebenfalls helixartige Form aufweist.

Aus Gründen der Übersichtlichkeit wurde hier auf die Darstellung von weiteren Aufstellelementen, Spannelementen und Beschichtungen eines weiteren, gegenüber dem ersten versetzten Rotorblattes verzichtet.

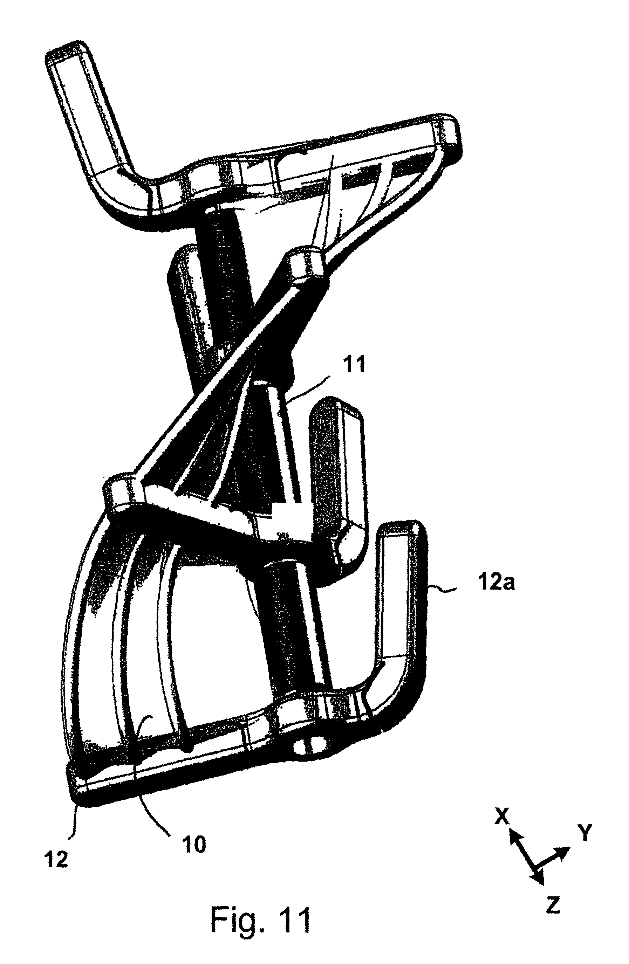

Die Fig. 11 zeigt einen Rotor mit einem einzigen Rotorblatt 10, das an vier Aufstellelementen 12 befestigt ist. Die den einzelnen Aufstellelementen 12 jeweils bezüglich der Welle 11 diametral gegenüberliegenden Aufstellelemente 12a tragen keine Membran und bleiben auch im Betrieb an die Welle 11 angeklappt, um Wirbelverluste in dem Fluid möglichst gering zu halten.

Auch hier wurden aus Gründen der Übersichtlichkeit Spannelemente und Beschichtungen nur einseitig dargestellt.
Die angeklappten Spannelemente sollen repräsentativ darstellen, wie der komplette Rotor mit angeklappten Elementen aussieht.

Die Fig. 12 zeigt in einer Draufsicht eine Rotorwelle 11, die sich im Betrieb in der durch den Pfeil 32 dargestellten Richtung dreht, wobei die Aufstellelemente 12, 13 derart profiliert sind, dass zu Beginn bei Inbetriebnahme der Pumpe bereits durch den Fluidgegendruck auf die Aufstellelemente eine radial nach außen gerichtete Kraft ausgeübt wird, die ein Abspreizen der Aufstellelemente 13, 14 von der Welle 11 bewirkt.

Sind die Aufstellelemente 12, 13 erst ein Stück weit abgespreizt, so beginnt auch die Membran zwischen ihnen, sich aufzustellen und einen Fluidgegendruck zu erzeugen, der rasch ein weiteres Aufstellen des Rotorblattes bewirkt, bis die Aufstellbewegung der Aufstellelemente durch einen mechanischen Anschlag oder die begrenzte Verformbarkeit der Aufstellelemente seine Grenzen findet.

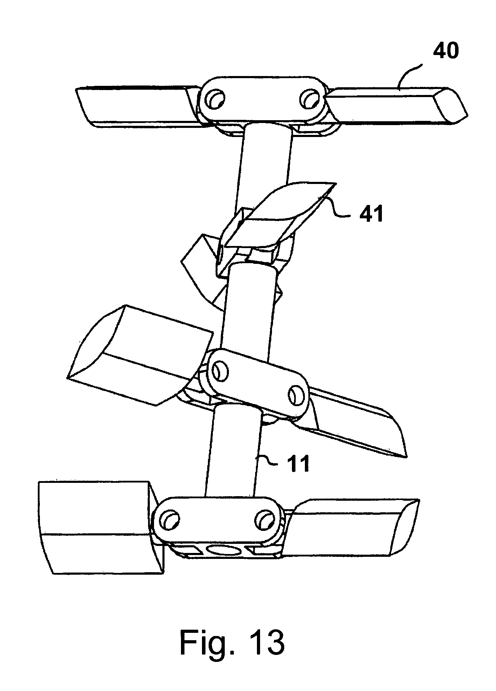

Figur 13 schließlich zeigt einen Rotor mit aufgestellten, propellerartigen Aufstellelementen 40,41, jedoch ohne Membran, in perspektivischer Ansicht.

Die erfindungsgemäße Gestaltung und das Fertigungskonzept der dargestellten Rotorblätter erlaubt mit geringem Aufwand die Herstellung von effizient einsetzbaren Rotorblättern mit Spannelementen und Membranen, die zuverlässig einsetzbar sind. Da die Rotorblätter bis auf die Aufstellelemente aus biegeschlaffen Materialien bestehen, ist eine hohe Kompressibilität des Rotors bei geringer Gegenkraft zu erreichen. Beim Aufstellen der Rotorblätter werden die Strömungseffekte bei Inbetriebnahme der Pumpe genutzt. Soll der Rotor wieder komprimiert werden, so bietet sich ein Rotieren des Rotors entgegen der Betriebsrichtung an, um die Aufstellelemente wieder an die Rotorwelle 11 anzuklappen.

## Patentansprüche

1. Fluidpumpe, insbesondere Flüssigkeitspumpe, mit einem Rotor (7), mit einer Rotorwelle (11) und wenigstens einem Rotorblatt zur Förderung des Fluids, wobei der Rotor bezüglich seines Durchmessers zwischen einem ersten, komprimierten Zustand und einem zweiten, expandierten Zustand veränderbar ist, wobei das wenigstens eine Rotorblatt wenigstens zwei entlang der Längsachse (18) der Rotorwelle (11) voneinander beabstandete Aufstellelemente (12,13,14)aufweist, die in expandiertem Zustand des Rotors von der Welle abstehen, sowie wenigstens zwei biegeschlaffe Spannelemente (25,26,27,28,29,30), die mit Abstand voneinander von einem Aufstellelement (12,13,14) wenigstens bis zu einem weiteren Aufstellelement verlaufen,
wobei zwischen den Spannelementen eine biegeschlaffe Membran (10) gehalten ist, welche in expandiertem Zustand des Rotors gespannt ist.

2. Fluidpumpe nach Anspruch 1, **dadurch gekennzeichnet, dass** die Aufstellelemente (12,13,14) jeweils mit einem ersten ihrer Enden an der Rotorwelle befestigt sind, wobei das jeweils andere Ende gegenüber der Rotorwelle beweglich ist.

3. Fluidpumpe nach Anspruch 2, **dadurch gekennzeichnet, dass** die Aufstellelemente (12,13,14) jeweils mit ihrem ersten Ende mit der Rotorwelle (11) schwenkbar verbunden sind.

4. Fluidpumpe nach Anspruch 3, **dadurch gekennzeichnet, dass** die Aufstellelemente (12,13,14) mit der Rotorwelle (11) jeweils mittels eines Lagers oder eines Filmgelenks verbunden sind.

5. Fluidpumpe nach Anspruch 2, 3 oder 4, **dadurch gekennzeichnet, dass** die Aufstellelemente (12,13,14) gegenüber der Rotorwelle (11) in deren Umfangsrichtung schwenkbar sind.

6. Fluidpumpe nach einem der Ansprüche 2, 3 oder 4, **dadurch gekennzeichnet, dass** die Aufstellelemente (12,13,14)gegenüber der Rotorwelle (11) in einer Ebene schwenkbar sind, die die Längsachse (18) der Rotorwelle enthält.

7. Fluidpumpe nach Anspruch 1 oder einem der folgenden, **dadurch gekennzeichnet, dass** die Aufstellelemente eine Form aufweisen, die bei einer Rotation des Rotors in Betriebsrichtung durch den Fluidgegendruck eine auf die Aufstellelemente wirkende, das Aufstellen der Aufstellelemente bewirkende Kraft erzeugt.

8. Fluidpumpe nach Anspruch 1 oder einem der folgenden, **dadurch gekennzeichnet, dass** die Aufstellelemente (12,13,14) entlang der Längsachse (18) der Rotorwelle (11) gegeneinander in Umfangsrichtung versetzt sind.

9. Fluidpumpe nach Anspruch 1 oder einem der folgenden, **dadurch gekennzeichnet, dass** die Membran (10) fest mit wenigstens zwei Spannelementen (25,26,27,28,29,30), insbesondere Spanndrähten, verbunden ist.

10. Fluidpumpe nach Anspruch 9, **dadurch gekennzeichnet, dass** die Spannelemente mit wenigstens zwei, insbesondere allen Aufstellelementen (12,13,14) zugfest verbunden sind.

11. Fluidpumpe nach Anspruch 1 oder einem der folgenden, **dadurch gekennzeichnet, dass** die Aufstellelemente (12,13,14) bis zu einem mechanischen Anschlag (23,24) begrenzt schwenkbar sind.

12. Fluidpumpe nach Anspruch 1 oder einem der folgenden, **dadurch gekennzeichnet, dass** die Spannelemente (25,26,27,28,29,30) im Wesentlichen parallel zueinander verlaufen.

13. Fluidpumpe nach Anspruch 12, **dadurch gekennzeichnet, dass** die Spannelemente (25,26,27,28,29,30) im Wesentlichen wendelförmig um die Rotorwelle (11) herum verlaufen.

14. Fluidpumpe nach Anspruch 1 oder einem der folgenden, **dadurch gekennzeichnet, dass** die Membran (10) an der Rotorwelle (11) dicht abschließt.

15. Verfahren zur Herstellung eines Rotors für eine Fluidpumpe nach einem der Ansprüche 1 bis 14, **dadurch gekennzeichnet, dass** durch Eintauchen der Spannelemente (25,26,27,28,29,30) in eine Flüssigkeit zwischen diesen eine Membran (10) gebildet wird, die sich nach dem Entfernen aus der Flüssigkeit verfestigt.

16. Verfahren zur Herstellung eines Rotors für eine Fluidpumpe nach einem der Ansprüche 1 bis 14, **dadurch gekennzeichnet, dass** zunächst die Spanelemente ausgerichtet werden und dass danach auf diesen eine Membran befestigt wird.
